# EUROPEAN PATENT APPLICATION

(11) **EP 3 578 129 A1**
(43) Date of publication of application: **11.12.2019**
(21) Application number: 19178569.0
(22) Date of filing: 05.06.2019
(51) Int. Cl.: A61B 50/33

(54) **SAMPLE RETRIEVAL KIT AND SYSTEM AND METHOD OF USE THEREOF**

(30) Priority: 05.06.2018 US 201862680720 P
(71) Applicant: Cook Medical Technologies LLC, Bloomington, IN 47404 (US)
(72) Inventor: O'CALLAGHAN, Rory, Co. Cavan (IE); CLANCY, Michael, Limerick (IE); NEILAN, John, Co. Galway (IE); DENNIS, Patrick, Dublin 3 (IE)
(74) Representative: Warren, Caroline Elisabeth

(57) **Abstract**

The present disclosure relates generally to systems and kits for the retrieval of samples from a biopsy needle and to methods of using such devices. In one embodiment, components of the system are supplied in the form of a kit contained in packaging that is utilized during the retrieval of the sample.

## Description

### TECHNICAL FIELD

The present disclosure relates to systems and kits for the retrieval of samples from a biopsy needle and to methods of using such devices to obtain such a sample from the body of a patient. In one embodiment, components of the system are supplied in the form of a kit contained in packaging that is utilized during the retrieval of the sample.

### BACKGROUND

Fine needle aspiration (FNA) is a diagnostic biopsy procedure used to obtain a sample from a target site in the body of a patient. A fine needle (for example 19-gauge to 25-gauge) is directed to a target site and suction is then applied to the proximal end of the lumen of the needle to aspirate a sample through its distal end. The procedure typically is far less invasive than other biopsy techniques, whether performed percutaneously (for example, to sample a suspected breast tumor or subcutaneous lesion) or endoscopically (for example, to sample a suspected cholangiocarcinoma via a duodenoscope). Moreover, advances in endoscopic ultrasound (EUS) technology have helped physicians and patients by providing enhanced ability of a physician to visualize a biopsy needle to obtain a sample of material from a target site without requiring an open incision or use of large-bore needles and/or laparoscopic trocars.

The method of sample retrieval from a patient is well defined with the physician adopting a consistent approach. However the method of collecting the sample from the biopsy needle is not consistent and many different methods may be utilized to remove the sample from the needle. Stylet advancement through the needle lumen, flushing with air or flushing with saline, or a combination of these methods can be required. This can lead to a considerable time delay in getting the needle back to the doctor to allow completion of the next needle pass.

Difficulty in removing the sample from the biopsy needle may result from multiple factors. Clotting of blood within the sample may make it difficult to advance the stylet through the biopsy needle. The consistency of the sample itself, for example, the presence of tissue within the sample, can also cause difficulties in advancing the stylet. In some cases, the needle cannula of the biopsy needle may take on an induced shape set that can make stylet advancement difficult.

A portion of the sample can be left within the lumen of the needle if the stylet diameter is smaller than the needle lumen size. A smaller stylet may be utilized to allow for a low stylet removal force, resulting in a portion of the sample remaining within the free space between the stylet diameter and the needle lumen diameter. In other instances, if air is used to expel the sample from the needle, the sample may dry and stick to the lumen wall of the needle.

In addition to the difficulties mentioned above, problems may arise in separating histology (tissue) and cytology (cells) materials in the sample expelled from the needle. The process of retrieving the sample from the biopsy needle and separating the histology and cytology portions of the sample may take a considerable time resulting in delays in the procedure and giving rise to an increased possibility of error. A consistent procedure for sample retrieval and separation would reduce the time required for the procedure and reduce the possibility of error.

### SUMMARY

Aspects of the invention are set out in the independent claims and preferred features are set out in the dependent claims. Preferred features of one aspect may be applied to other aspects alone or in combination with other aspects.

Certain aspects of the present invention provide kits and systems that may be utilized to provide a consistent method of retrieving both histology and cytology samples from a biopsy needle and for the separation of the components of such samples. One embodiment provides a kit containing components including a biopsy needle and a needle guide. In one embodiment, the kit includes a base element having a top surface provided with a biopsy needle mounting fixture, a collection container mounting fixture and a plurality of receptacles. The top surface may include a first and a second side wall extending upwards from the top surface, and a front edge and a back edge. A cover may be hinged to the back edge and shaped to extend over the first and second side walls to the front edge and to close the top surface. Printed instructions describing the sample retrieval process may be present on an inside surface of the cover. The base element may be, for example, a vacuum formed or molded element.

In one embodiment, the first and the second side walls each slope towards the front and the back edge of the base element. In another embodiment, the front edge includes a sealing lip for engaging the cover. The biopsy needle may be accommodated in one of the plurality of receptacles and the needle guide accommodated in another of the plurality of receptacles. A biopsy needle sheath catch may be positioned on the top surface and provide for the attachment of a portion of the biopsy needle sheath.

In one embodiment, the needle guide includes an elongated member having an interior cavity extending from an open upper end to an open lower end. In such an embodiment, a needle guide capping member including an access port is attached over the open upper end.

In another embodiment, a sample retrieval dart is positioned within the interior cavity of the needle guide near the open upper end. The sample retrieval dart includes a dart body having a dart lumen in-line with the access port and providing access between the open upper end and the interior cavity at the open lower end. In one embodiment, a non-return valve is positioned within the dart body in-line with the dart lumen. A needle guide sheath is positioned in the dart lumen below the non-return valve and extends downwards from the dart body into the interior cavity at the lower end. In another embodiment, the open lower end includes a sample container engagement member.

The kit may also include a collection container having a base sized to engage the collection container mounting fixture and a top opening sized to engage the sample container engagement member of the needle guide. The sample collection container may include a sample separation filter positioned between the base of the container and the top opening. The sample separation filter may be detachable from the sample collection container and may include a mesh filter, a shaped surface allowing for fluid run-on into the sample collection container or a folded filter paper. In other embodiments, the sample separation filter may be incorporated into the needle guide.

In other embodiments, the kit may include at least one of a vacuum syringe, a wash syringe, a collection container and a dislodge tool. These component(s) may be accommodated in the plurality of receptacles.

In one embodiment, the biopsy needle mounting fixture is sized and located to accept at least a portion of the handle of the biopsy needle and to position a proximal end of the biopsy needle handle beyond the front edge of the base element.

In another embodiment, the kit also includes a pump station. The pump station may include a syringe mounting fixture and a syringe plunger activation unit. In one embodiment, the syringe plunger activation unit includes an activation member movable to engage a plunger of a syringe mounted in the syringe mounting fixture and to move the plunger to expel fluid from a needle hub of the syringe, and a handle operatively connected to the activation member.

### BRIEF DESCRIPTION OF THE DRAWINGS

Figure 1 is an illustration of one embodiment of a kit including components useful in the collection and analysis of biopsy samples.
Figure 2(A) is a schematic cross section view of a base element of a container for use with the kits and systems of the present embodiments.
Figure 2(B) is a view looking toward the front edge of the base element of Figure 2(A).
Figure 2(C) shows a view of the base element and attached cover as viewed towards a side wall of the container.
Figure 2(D) shows a view in the same direction as in Figure 2(C) but through an axis of the biopsy needle mounting fixture.
Figure 3 shows the major components of one embodiment of a biopsy needle suitable for use with the kits and systems disclosed herein.
Figure 4(A) shows a side of one embodiment of a needle guide for use with a kit or system as disclosed herein
Figure 4(B) shows a top view to the needle guide of Figure 4(A).
Figure 5(A) shows an exploded view of the needle guide of Figure 4(A).
Figure 5(B) shows one embodiment of a filter unit for use with a kit including components useful in the collection and analysis of biopsy samples.
Figure 6(A) shows an illustration of one embodiment of a kit including components useful in the collection and analysis of biopsy samples. Here, a biopsy needle is shown positioned in a biopsy needle mounting fixture. A collection container is positioned in a collection container mounting fixture.
Figure 6(B) illustrates the proximal end of a biopsy needle extending from the biopsy needle mounting fixture.
Figure 6(C) is an illustration of the use of a dislodge tool to clear a blockage in a biopsy needle.
Figure 7(A) shows one embodiment of a dislodge tool.
Figure 7(B) shows a cross-sectional view of one embodiment of a dislodge tool. The dislodge tool is shown positioned within the needle guide so as to dislodge sample from the lumen of a biopsy needle.
Figure 8 shown another embodiment of a kit including components useful in the collection and analysis of biopsy samples.

### DETAILED DESCRIPTION OF THE PREFERRED EMBODIMENTS

### Definitions

For purposes of promoting an understanding of the present invention, reference will now be made to the embodiments illustrated in the drawings, and specific language will be used to describe the same. It should nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

In the following discussion, the terms "proximal" and "distal" will be used to describe the opposing axial ends of the biopsy needle assembly and associated components of the kit and system, as well as the axial ends of various component features of these devices. The term "proximal" is used in its conventional sense to refer to the end of the assembly (or component) that is closest to the medical professional during use of the assembly. The term "distal" is used in its conventional sense to refer to the end of the assembly (or component) that is initially inserted into the patient, or that is closest to the patient during use. The term "longitudinal" will be used to refer to an axis that aligns with the proximal-distal axis of the assembly or component. The term "lateral" will be used to refer to an axis or plane that is perpendicular to the proximal-distal axis of the assembly or component.

The uses of the terms "a" and "an" and "the" and similar references in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context.

As used herein the terms "comprise(s)," "include(s)," "having," "has," "can," "contain(s)," and variants thereof, are intended to be open-ended transitional phrases, terms, or words that do not preclude the possibility of additional acts or structures. The present invention also contemplates other embodiments "comprising," "consisting of' and "consisting essentially of," the embodiments or elements presented herein, whether explicitly set forth or not.

Kits for use in the collection of biopsy samples.

### Container and Workstation

Certain aspects of the present invention provide systems and kits including components useful in the collection, retrieval and analysis of biopsy samples. Preferably the biopsy samples are samples collected from a human or veterinary patient using a fine needle aspiration procedure. The present kits and systems may be utilized to provide a consistent method of retrieving both histology and cytology samples from the biopsy needle and for the separation of such samples. Such a method allows for considerable time saving during the procedure, provides samples of a consistent quality and allows the biopsy needle to be quickly returned to the physician performing the biopsy.

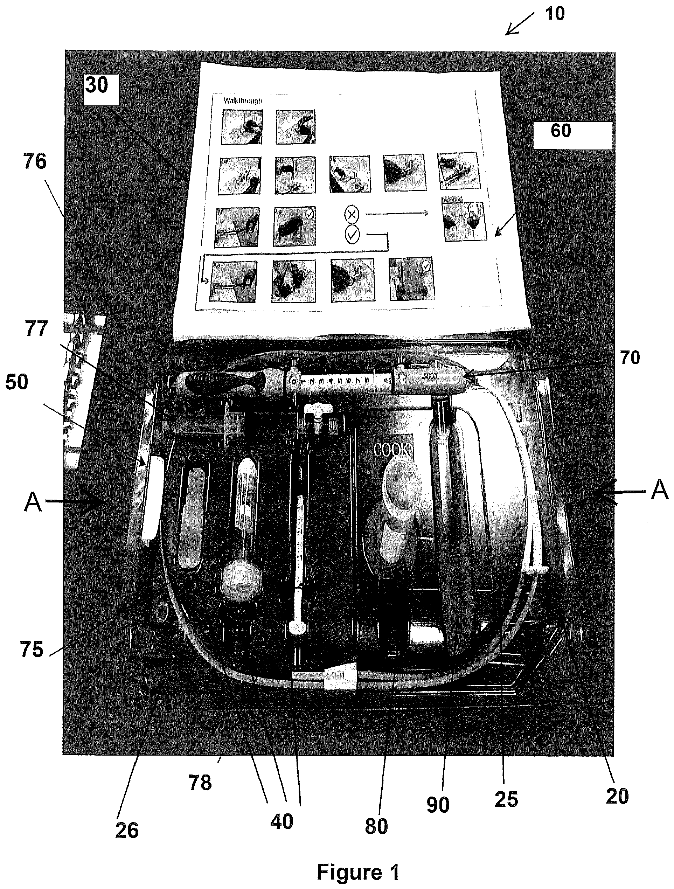

Turning first to Figure 1, there is here illustrated one embodiment of such a kit. Kit 10 includes a container with a base element 20 having a top surface 25 including a number of receptacles 40. The top surface may be utilized as a workstation during the sample collection and retrieval process. Cover 30 is preferably hinged to the back edge of base element 20 and is shaped to extend over and close top surface 25. The front edge of base element 20 may include a sealing lip for engaging the cover. In some embodiments, the inside surface of cover 30 includes printed instructions which can be reviewed by the operator during the sample collection and retrieval procedure. The base element 20 may be, for example, a vacuum formed or a molded element.

Receptacles 40 are shaped and sized to contain various components used in the sample retrieval procedure. Such components may include biopsy needle 70 and needle guide 75. Receptacles 40 may also include at least one additional component, such as a vacuum syringe 77, a wash syringe 78, a collection container or a dislodge tool 76. The purpose of such additional components is described in more detail below. In certain embodiments, at least one receptacle may contain a sample collection container. Top surface 25 may also include a biopsy needle mounting fixture 90 and a collection container mounting fixture 80.

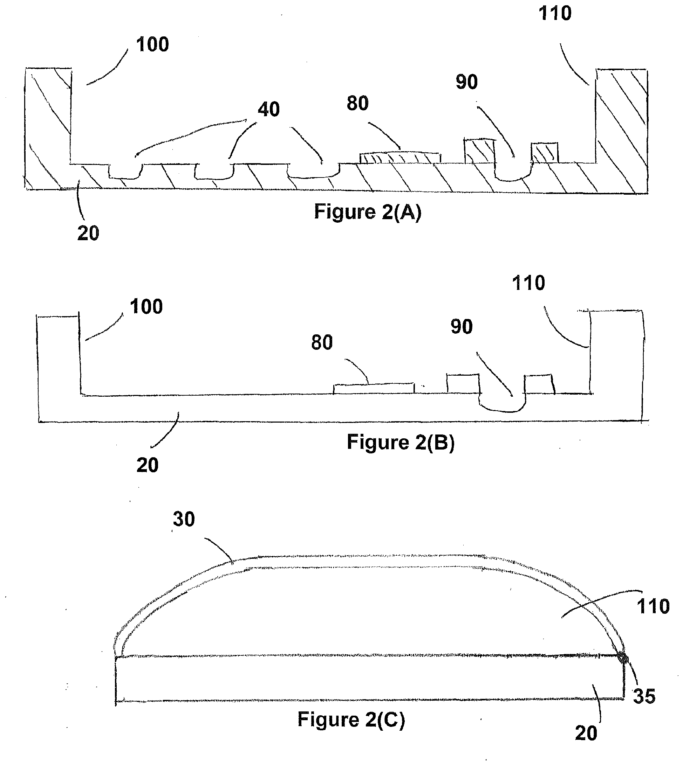

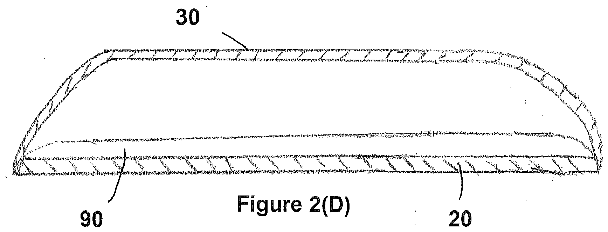

Turning now to Figures 2(A-D). Figure 2(A) shows a cross sectional view of base element 20 along axis A-A (Figure 1). Here, top surface 25 includes biopsy needle mounting fixture 90 and collection container mounting fixture 80, as well as receptacles 40. Side walls 100 and 110 extend upwards at the sides of top surface 25. Preferably at least one of the front edge and the back edge of base element 20 does not extend upwards from top surface 25 (i.e. is level with top surface 25) or extends upwards from top surface 25 only to a degree that allows a biopsy needle positioned horizontally or substantially horizontally in biopsy needle mounting fixture 90 to extend beyond at least one of the front edge and the back edge of base element 20.

Figure 2(B) is a view looking toward front edge 26 of base element 20. Here, the front edge and back edge of base element 20 do not include a wall extending upwards from top surface 25 and a biopsy needle positioned as described in biopsy needle mounting fixture 90 may, depending on its length, extend beyond the front and/or the back edge of base element 20.

Figure 2(C) shows a view of base element and attached cover 30 as viewed towards side wall 110. Here, it can be seen that cover 30 attaches to base element 20 at hinged join 35 and extends over side wall 110 (and top surface 25) to the front edge of base element 20. In one embodiment, the first and the second side walls each slope towards the front and the back edge of the base element as is illustrated in Figure 2(C).

Figure 2(D) shows a view in the same direction but through an axis of biopsy needle mounting fixture 90. Here, it can be seen that, when cover 30 is removed from over top surface 25, when the biopsy needle is positioned in biopsy needle mounting fixture 90 it may extend beyond the front and/or back edge of base element 20.

### Biopsy needle

The system and/or kit as disclosed herein may include a variety of biopsy needle designs. The biopsy needle may be, for example, one of the biopsy needles disclosed in US Patent Publication Number 2014/0005478, published January 2, 2014, or in US Patent Publication Number 2017/0156709, published July 8, 2017, the contents of which are incorporated by reference.

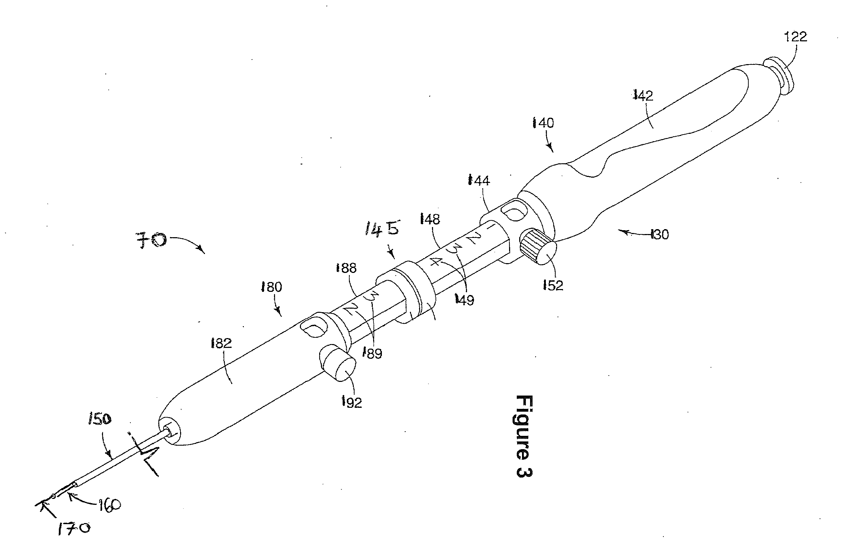

Referring now to Figure 3, the major components of one embodiment of a biopsy needle are described. Biopsy needle 70 includes a handle 130, a sheath 150 extending distally from the handle 130, an elongate flexible cannula 160 positionable within the sheath, and a stylet 170 positionable with the lumen of cannula 160. Handle 130 includes a proximal handle segment 140, a dividing region 145, and a distal handle segment 180.

As shown in Figure 3, the proximal handle segment 140 preferably includes a gripping section 142 and a cannula adjuster 144. The cannula adjuster 144 is positioned distal to the gripping section 142 and may be configured (include an identifier) to identify the length of the extension of the flexible cannula 160 out of the sheath 150. The cannula adjuster 144 may move along a proximal shaft 148 of the handle 130, and may include indicia 149 relating to the length that the flexible cannula 160 extends out of the sheath 150. A locking mechanism 152 may be used to releasably lock the cannula adjuster 144 in position. For example, the cannula adjuster 144 being positioned at "0" indicates that the flexible cannula 160 is positioned within the sheath 150 for insertion through an endoscope. By contrast, the cannula adjuster 144 being positioned at "3" or "4" indicates a predetermined amount of extension of the flexible cannula 160 beyond a distal end of the sheath 150. Figure 3 shows flexible cannula 160 extending slightly out of sheath 150.

The distal handle segment 180 comprises a sheath adjuster 182 that is movable over a distal shaft 188 having measurement indicia 189. A locking mechanism 192 may be used to releasably lock the sheath adjuster 182 in position relative to the distal shaft 188. The sheath adjuster 182 may be advanced proximally or distally relative to the sheath 150 when the locking mechanism 192 is disengaged, thereby allowing the sheath adjuster 182 to slide over the distal shaft 188 and relative to the sheath 150, which helps makes the biopsy needle 70 compatible with a full range of endoscopes. In some embodiments, the distal handle segment 180 is separated from the proximal handle segment 140 by the dividing region 145.

The elongate flexible cannula 160 of the biopsy needle 70 includes a cannula lumen extending between a distal end and a proximal end of the cannula 160. The flexibility of at least a portion of the elongate flexible cannula 160 preferably provides pushability and trackability sufficient to allow navigation through a body lumen or other passage without significant risk of crimping or otherwise occluding the cannula lumen. In one embodiment, the elongate flexible cannula 160 may be constructed of stainless steel hypotube or a nickel-titanium alloy. As another example, one embodiment may include a polyether block amide (PEBA), PEBAX, poly-ether-ether-ketone (PEEK), ePTFE, PTFE, or PET cannula, and it will be appreciated that other polymeric materials including polymers with braided construction and/or with metallic components may also be used within the scope of this invention. This pushability and trackability will be enhanced by the removable stylet 170.

Removable stylet 170 may pass through and extend distally from the cannula lumen of the cannula 160. The stylet 170 may include a piercing tip, which may be provided in a lancet configuration or any number of other beveled configurations which will include any design configured for effectively piercing tissue. The tissue may be, by way of illustrative example, the exterior of a pancreatic pseudocyst, the wall of the stomach, an intestinal wall, or another artificial or natural structure between an endoscopically-accessible site and a target site, including creation of an orifice for a natural orifice translumenal endoscopic (NOTES) surgical procedure. The stylet 170 extends longitudinally through the elongate flexible cannula 160 to provide stiffness to the elongate flexible cannula 160 before piercing and/or sampling of the tissue.

The stylet 170 may include an echogenic surface, and may be made from any material known in the art. By way of example, and without limitation, materials for the stylet 170 may include stainless steel, a memory-metal alloy, such as nickel titanium, a composite, and/or similar alloys. The stylet 170 also may be constructed of and/or coated with a polymer including, for example, an echogenic polymer.

Referring still to Figure 3, an exemplary operation of the biopsy needle 70 will be described. The cannula adjuster 144 is positioned at "0" which indicates that the flexible cannula 160 and also the stylet 170 are positioned within the sheath 150 for insertion through an endoscope. A distal region of the endoscope is advanced down through a bodily lumen to a target tissue site. The distal end of the endoscope is maneuvered in close proximity to the target tissue. The sheath 150 then is loaded into a proximal end of the endoscope through an accessory channel of the endoscope. The sheath 150 then is advanced until it is near the distal end of the accessory channel.

In a next step, the cannula adjuster 144 and the gripping section 142 may be advanced distally relative to the proximal shaft 148. For example, the cannula adjuster 144 may be positioned at increment "4", indicating that the flexible cannula 160 extends a predetermined amount beyond a distal end of the sheath 150. Additionally, the stylet 170 may extends distally beyond the distal end of the flexible cannula 160 at this time.

Locking mechanism 152, for example, a thumbscrew, is part of the cannula adjuster 144 and selectively applies pressure to proximal shaft 148. Movement of the proximal handle 140 can selectively move the proximal handle 140 forward as far as the adjuster 144. A proximal portion of cannula 160 attaches to proximal handle 140 and is also moved distally. The echogenic features of the flexible cannula 160 and/or the stylet 170, described above, allow for enhanced visualization near the target tissue site. Using such enhanced visualization, the flexible cannula 160 and the stylet 170 are advanced distally such that the lancet point of the stylet 170 pierces the target tissue.

Still referring to Figure 3, in a next step, the stylet 170 may be retracted proximally by pulling on a proximal knob 122. Through one or more of suction, rotary manipulation, and/or longitudinal manipulation of the needle cannula 160, the cannula will excise and capture tissue, which preferably will include sufficiently intact samples for histology and/or cytology, from the target site through the distal end of needle cannula 160. The needle may then be withdrawn from the patient's body, preferably after the distal end of needle cannula 160 is returned to the lumen of sheath 150.

### Needle Guide

Various exemplarily embodiments of the needle guide will now be described. The function of the needle guide is to accept the distal end of the biopsy needle sheath and the needle cannula contained within, and to provide a mechanism for the retrieval of the sample from the lumen of the needle cannula. Preferably the needle guide is a single use component that is discarded after retrieval of the sample from the biopsy needle.

In certain embodiments, the needle guide will be attached to the top of a collection container positioned in the collection container mounting fixture 80. The distal end of the sheath of the biopsy needle is inserted into a port in the needle guide and is accommodated within a needle guide lumen. The needle guide may include a non-return valve positioned in line with the needle guide lumen. In some embodiments, the distal end of the biopsy needle sheath is firmly held at or in the region of the non-return valve and the cross-section dimension of the needle guide lumen reduced at this point such that only the cannula, but not the access sheath, may extend beyond the reduced dimension into the lower region of the needle guide lumen.

The needle guide provides a mechanism for retrieval of the sample from the biopsy needle cannula without exposing the distal end of the needle cannula so as to prevent the risk of a needle stick type injury to the operator. The distal end of the needle cannula remains contained within the biopsy needle sheath until the sheath is inserted into the needle guide. Only then is the cannula extended from the sheath to allow for retrieval of the sample.

In other embodiments, the needle guide may include additional components that enhance the functionality of the needle guide. One embodiment provides a mechanism allowing for the separation of the histological and cytology components of the retrieved sample. For example, the needle guide may include a filter positionable between needle guide lumen and the collection container. When the sample is expelled from the needle cannula, the histological sample is retained in the filter whereas the cytological component passes through the filter into the collection container. The filter may be, for example, a mesh filter, a shaped surface allowing for fluid run-on into the container or a folded filter paper.

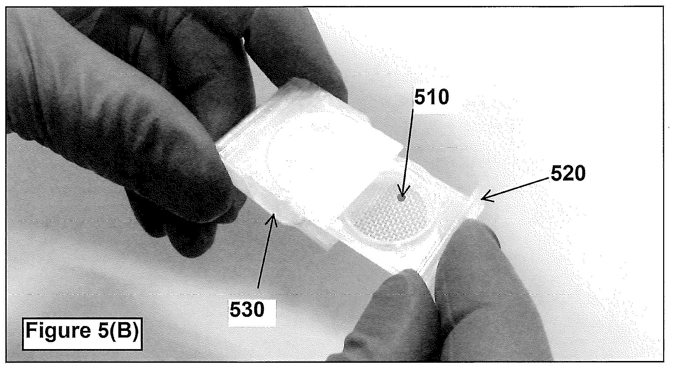

One embodiment of such a filter is illustrated in Figure 5(B). Here, filter 510 is contained in a holder 520 sized and shaped for slidably or otherwise engaging the lower portion of the needle guide between the distal end of the needle guide cannula and the collection container mounting fixture such that sample expelled from the needle guide cannula is deposited on the surface of the filter. Holder 520 may be provided with a detachable cap 530 movable between a first position against one edge of the holder (as illustrated) and a second position wherein the holder is contained within the detachable cap. In other embodiments, the holder may be provided with a hinged lid allowing for exposure of a top surface of the filter when the folder is positioned within the needle guide.

In other embodiment, the sample separation filter unit is not included in the needle guide but is instead incorporated into the sample collection container. For example, the filter unit may be positioned between the base of the sample collection container and the top opening of the sample collection container. The sample separation filter may be detachable from the sample collection container and may be provided with a detachable cap to protect any histology sample contained on the filter surface.

In another embodiment, the needle guide includes a wash station allowing for the distal end of the needle cannula to be washed with either air, water or another fluid to assist in the removal of sample from the needle cannula.

In various embodiments, the needle guide provides for retrieval of the sample from the needle cannula by allowing positioning the needle when either applying a vacuum at the distal end of the needle cannula or blowing the sample from the lumen of the needle cannula by applying a fluid at pressure to the proximal end of the biopsy needle cannula. If vacuum is to be utilized, a vacuum port may be incorporated into the needle guide and, for example, a region below the distal end of the needle cannula may be sealable to allow for the maintenance of a reduced pressure to expel the sample.

Alternatively, the sample may be expelled by delivering a fluid, for example air, water or saline, at pressure to the proximal end of the needle cannula (for example, at the proximal end of biopsy needle handle 140.) In such embodiments the kit and/or system may include a pump mechanism for delivering such a fluid. In some embodiments, such a pump is supplied in one of the receptacles of the base element. In other embodiments, such a pump is supplied as a separate component in its own packaging. In other embodiments, the fluid may be delivered utilizing a syringe, for example the wash syringe.

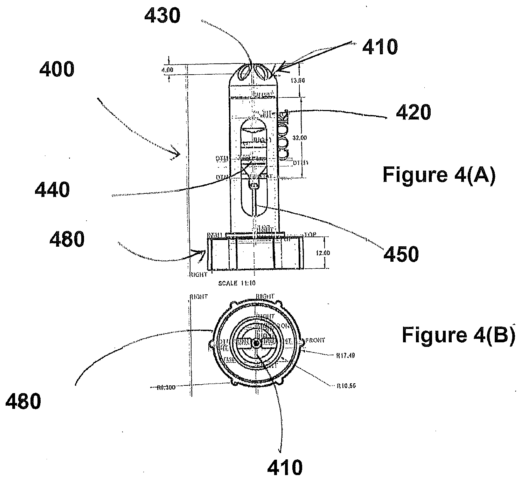

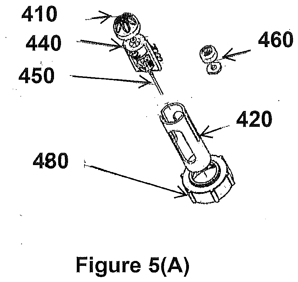

Turning now to Figures 4(A-B) and Figure 5(A), there is here illustrated one embodiment of a needle guide for use with various embodiments of the kit and system as disclosed herein. Figure 4(A) shows a side view of needle guide 400 while Figure 4(B) shows a top view to the needle guide. Figure 5(A) shows an exploded view illustrating the internal components of one embodiment of the needle guide. Needle guide 400 includes an elongated member 420 having an interior cavity extending from an open upper end to an open lower end. Needle guide capping member 410 positioned over the open upper end and includes access port 430 providing access to the internal cavity. Sample retrieval dart 440 is positioned in the interior cavity near the upper end and includes a dart body having a dart lumen providing access between the open upper end and the interior cavity at the open lower end. The dart lumen is in-line with the access port 430 and is sized to accept the sheath of the biopsy needle. In some embodiments, non-return valve 460 is positioned within the dart body in-line with the dart lumen. In such embodiments, the biopsy needle sheath may be positioned at the non-return valve to prevent backflow of the sample around the exterior of the needle sheath when the sample is retrieved from the cannula lumen of the biopsy needle.

Needle guide sheath 450 is positioned in the dart lumen below and in-line with the non-return valve and extends downwards from the dart body into the interior cavity at the lower end. The lumen of needle guide sheath 450 is sized to accept the cannula of the biopsy needle then it is moved beyond the distal end to the biopsy needle sheath. In some embodiments, the size of needle guide sheath 450 below the non-return valve is such that the cannula and not the biopsy needle sheath is accommodated by sheath 450. In other embodiments, sheath 450 is transparent or at least translucent, allowing the operator to observe the biopsy needle cannula within sheath 450 if elongated member 420 includes an open or windowed region. For example, sheath 450 may be formed from glass or a polymer, such as polycarbonate. In other embodiments, sheath 450 is opaque.

In one embodiment, the inner diameter of the needle guide sheath below the non-return valve closely matches the outer diameter of the biopsy needle cannula such that exit of the sample through a side notch of the biopsy needle cannula is prevented.

The lower end of elongated member attaches to sample container engagement member 480. Engagement member 480 includes a lower region sized and shaped to engage the top rim of a collection container. Such a collection container is typically positioned in collection container mounting fixture 80 and needle guide 400 attached to the top of the container by engagement member 480. With the distal end of the biopsy needle sheath inserted into the dart lumen, the biopsy needle cannula may be moved distally out of the needle sheath and positioned in the dower region of needle guide sheath 450.

### Kit including a Pump Station

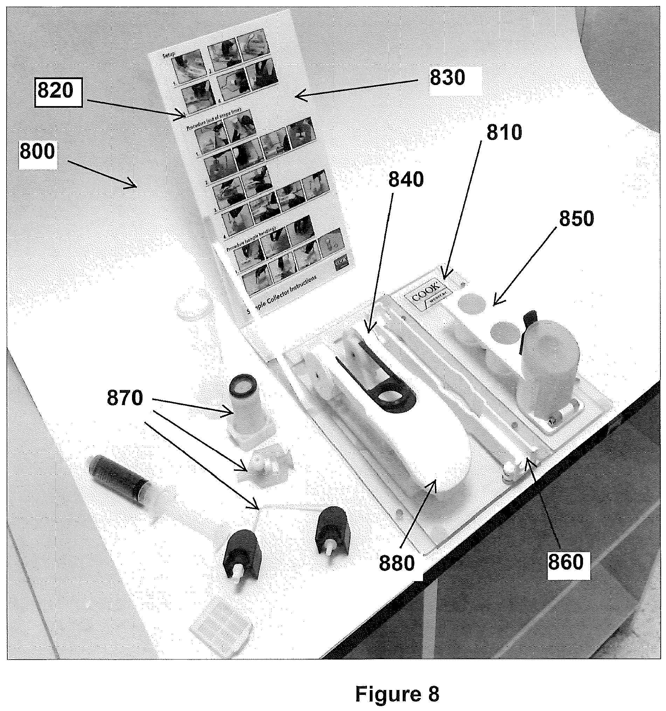

Turning now to Figure 8, there is here illustrated another embodiment of a kit including components useful in the collection and analysis of biopsy samples. Here, kit 800 includes a container including a base element 810 having a top surface that may be utilized as a workstation during the sample collection process. Cover 830 is preferably hinged to the back edge of base element 810 and is shaped to extend over and close the top surface. In some embodiments, the inside surface of cover 830 includes printed instructions 820 which can be reviewed by the operator during the sample retrieval procedure. Base element 810 may be, for example, a vacuum formed or molded element.

Biopsy needle mounting fixture 860 and collection container mounting fixture 850 are present on the top surface of base element 810. Here, sample container mounting fixture 850 may include multiple positions for accommodating different sized containers, including, for example, sample collection and/or centrifuge containers.

In one embodiment, pump 840 is mounted on base element 810 and includes a syringe plunger activation unit 880 comprising an activation member movable to engage a plunger of a syringe mounted in a syringe mounting fixture and to move the plunger to expel fluid from a needle hub of the syringe, and a handle operatively connected to the activation member. In other embodiments, the pump is provided in separate packaging and need not be positioned on the top surface of base element 810 during the sample retrieval process.

Additional disposable components 870 including connectors for attaching the needle hub of the syringe to the biopsy needle, a needle guide, filter unit, collection container, wash syringe and dislodge tool may be provided in separate packaging or may be provided in receptacles on the top surface of base element 810.

### Methods of retrieving a biopsy sample

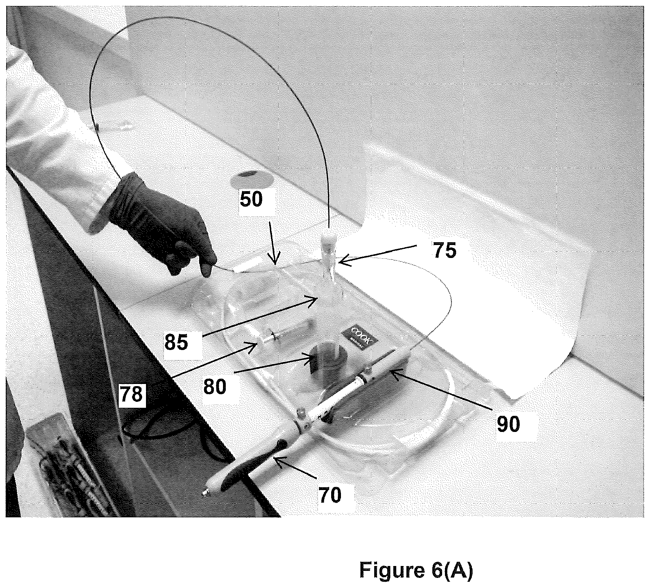

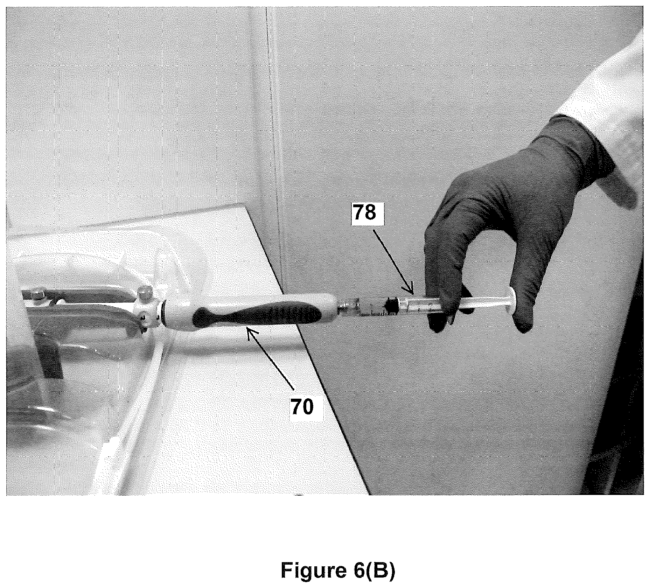

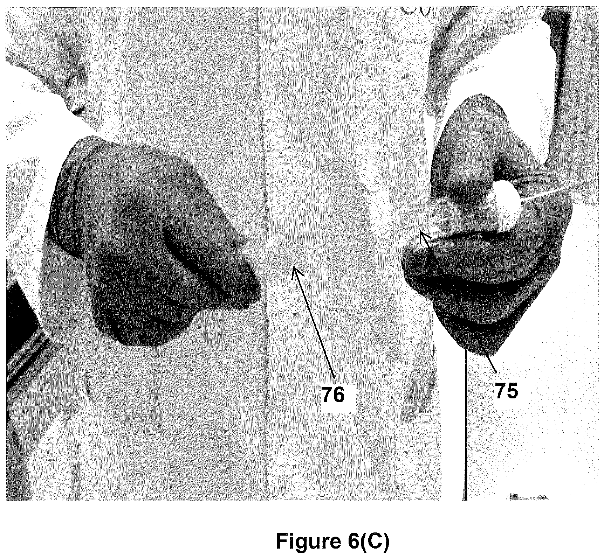

One embodiment of a method of retrieving a biopsy sample utilizing kits and systems as disclosed herein will now be described with reference to Figures 6(A-C). The sample is collected from the patient using, for example, an EUS/FNA Biopsy Needle as described herein. Sample collection may be, but need not be, performed using a biopsy needle supplied as a component of a kit or system as disclosed herein. In some embodiments, a vacuum syringe may be supplied as a component of the kit or system and be connected to the distal end of the biopsy needle and used to draw the sample into the needle cannula.

With the sample in the needle cannula, the operator will bring the device to where the base unit of the kit or system is positioned. Prior to sample retrieval, the operator will insert a collection container (such as a container commonly used for cytology/histology samples) into the collection container mounting fixture of the base element and position the needle guide in place on top of the collection container.

The handle of the biopsy needle is then inserted into the biopsy needle mounting fixture and the biopsy needle sheath inserted into the access port of the needle guide capping member. Figure 6(A) shows a collection container 85 positioned in collection container mounting fixture 80 with needle guide 75 in place on the sample container. Wash syringe 78 is shown positioned in one of the receptacles present on the top surface of the base unit.

Biopsy needle 70 is shown positioned in biopsy needle mounting fixture 90. Here, the proximal end of biopsy needle 70 extends from the front edge of the base unit and the sheath of the biopsy needle extends from the back edge of the base unit. The absence of high front and back walls in the base unit assists in the positioning of the biopsy needle, allowing for access to the proximal and distal ends of the biopsy needle handle. The needle sheath is shown looped under the biopsy needle sheath catch 50 positioned on the top surface of the base element.

The biopsy needle is then unlocked and the handle is pushed forward, extending the biopsy needle cannula into the sheath of the needle guide as disclosed above. In this embodiment, a wash syringe, such as wash syringe 78, is primed and attached and the proximal end of biopsy needle 70 and the sample is flushed as shown in Figure 6(B). In some embodiment, a filter unit as disclosed herein in positioned in the lower of the needle guide to collect any tissue included in the sample and to separate the histology and cytology portions of the sample. If a sample is observed in the collection container, the procedure continues as normal. However, if a sample is not observed, the operator may remove needle guide 75 from the collection container and attempt to dislodge the needle using a dislodge tool, such as dislodge tool 76, as is illustrated in Figure 6(C).

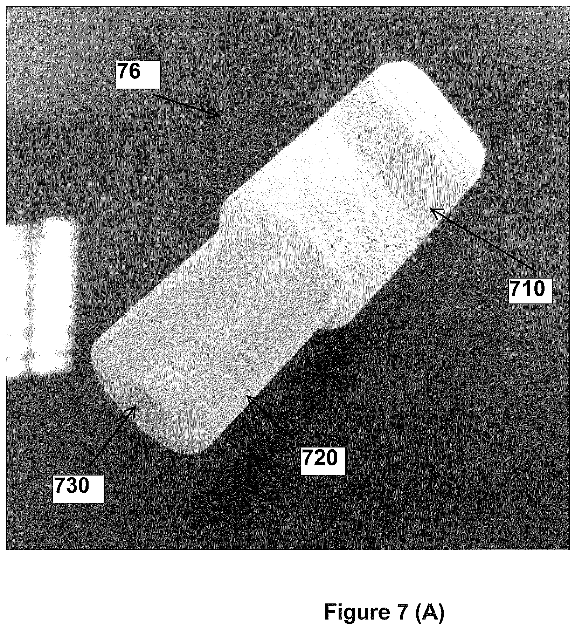

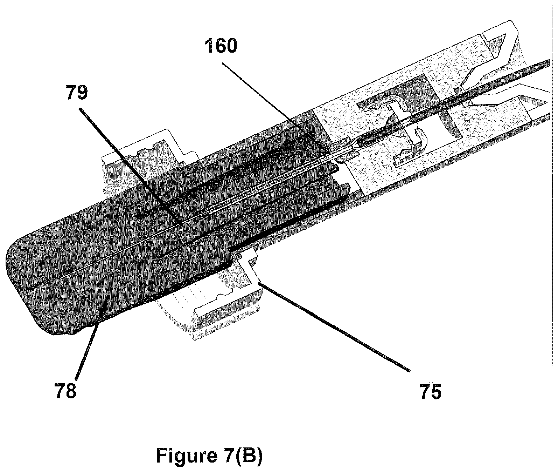

Figure 7(A) illustrates one embodiment of such a dislodge tool. Dislodge tool 76 includes a grip portion 710 and a barrel portion 720. Barrel portion 720 is sized and shaped to fit into the lower portion of the needle guide and includes lumen 730 which accepts the lower portion of the needle guide sheath. Figure 7(B) shows a cross-sectional view of dislodge tool 76 positioned within needle guide 75 so as to dislodge sample from the lumen of a biopsy needle cannula 160.

After the sample is delivered into the collection container, the needle may be flushed with air to ensure the channel is clear and the stylet reinserted. The cannula of the biopsy needle may then be withdrawn from the needle guide sheath and into the biopsy needle sheath. The needle is then locked and is again ready for use.

For the avoidance of doubt, it will be appreciated that the method of retrieving the biopsy sample occurs outside the body and is practiced on the biopsy needle and not on the body itself.

### Summary of embodiments

In summary, embodiments of the present disclosure relate generally to systems and kits for the retrieval of samples from a biopsy needle and to methods of using such devices. In one embodiment, components of the system are supplied in the form of a kit contained in packaging that is utilized during the retrieval of the sample.

Those of skill in the art will appreciate that embodiments not expressly illustrated herein may be practiced within the scope of the present invention, including that features described herein for different embodiments may be combined with each other and/or with currently-known or future-developed technologies while remaining within the scope of the claims presented here. It is therefore intended that the foregoing detailed description be regarded as illustrative rather than limiting. And, it should be understood that the following claims, including all equivalents, are intended to define the spirit and scope of this invention. In the event of any inconsistent disclosure or definition from the present application conflicting with any document incorporated by reference, the disclosure or definition herein shall be deemed to prevail.

Exemplary embodiments of aspects of the system are set out below in the following numbered clauses:
1. A kit comprising:
   (a) a biopsy needle;
   (b) a needle guide; and
   (b) a container comprising
   a base element comprising a top surface provided with a biopsy needle mounting fixture, a collection container mounting fixture and a plurality of receptacles, wherein the top surface comprises a front edge, a back edge,
   a first side wall and a second side wall each extending upwards from the top surface; and
   a cover hinged to the back edge and shaped to extend over the first and second side walls to the front edge and to close the top surface,
   wherein the biopsy needle is accommodated in one of the plurality of receptacles and wherein the needle guide is accommodated in another of the plurality of receptacles.
2. The kit of clause 1, wherein the first and the second side walls each slope towards the front and the back edge of the base element and wherein a sealing lip for engaging the cover is positioned on the front edge.
3. The kit of clause 1 or 2, wherein the needle guide comprises:
   an elongated member having an interior cavity extending from an open upper end to an open lower end;
   a needle guide capping member positioned over the open upper end, the needle guide capping member having an access port;
   a sample retrieval dart positioned within the interior cavity near the open upper end, and comprising a dart body having a dart lumen providing access between the open upper end and the interior cavity at the open lower end, wherein the dart lumen is in-line with the access port;
   a non-return valve positioned within the dart body, in-line with the dart lumen, and a needle guide sheath positioned in the dart lumen below the non-return valve and extending downwards from the dart body into the interior cavity at the lower end, wherein the open lower end comprises a sample container engagement member.
4. The kit of clause 1, 2, or 3, further comprising a biopsy needle sheath catch positioned on the top surface.
5. The kit of any preceding clause, further comprising a pump station comprising:
   a syringe mounting fixture, and
   a syringe plunger activation unit comprising a activation member movable to engage a plunger of a syringe mounted in the syringe mounting fixture and to move the plunger to expel fluid from a needle hub of the syringe, and a handle operatively connected to the activation member.
6. The kit of any preceding clause, further comprising at least one component selected from the group consisting of: a vacuum syringe, a wash syringe, a collection container and a dislodge tool, wherein the at least one component is accommodated in the plurality of receptacles.
7. The kit of any preceding clause, wherein the biopsy needle mounting fixture is sized and located to accept a handle of the biopsy needle and to position a proximal end of the biopsy needle handle beyond the front edge of the base element.
8. The kit of clause 1, further comprising a sample collection container having a base sized to engage the collection container mounting fixture and a top opening sized to engage the sample container engagement member of the needle guide.
9. The kit of clause 8, wherein the sample collection container further comprises a sample separation filter positioned between the base and the top opening, said sample separation filter optionally being detachable from the sample collection container.
10. The kit of clause 9, wherein the sample separation filter comprises a filter selected from the group consisting of a mesh filter, a shaped surface allowing for fluid run-on into the sample collection container, and a folded filter paper.
11. The kit of clause 1, further comprising a centrifuge container mounting fixture on the top surface.
12. The kit of clause 1, wherein the needle guide further comprises a sample separation filter positioned between the needle guide sheath and the sample container engagement member, and further comprising a sample collection container, wherein the sample separation filter optionally is detachable from the needle guide.
13. The kit of clause 12, wherein the sample separation filter comprises a filter selected from the group consisting of a mesh filter, a shaped surface allowing for fluid run-on into the sample collection container, and a folded filter paper.
14. A needle guide comprising:
   an elongated member having an interior cavity extending from an open upper end to an open lower end;
   a needle guide capping member positioned over the open upper end, the needle guide capping member having an access port;
   a sample retrieval dart positioned in the interior cavity near the open upper end, and comprising a dart body having a dart lumen providing access between the open upper end and the interior cavity at the open lower end, wherein the dart lumen is in-line with the access port;
   a non-return valve positioned within the dart body in line with the dart lumen, and a needle guide sheath positioned in the dart lumen below the non-return valve and extending downwards from the dart body into the interior cavity at the lower end, wherein the open lower end comprises a sample container engagement member.
15. The needle guide of clause 14, further comprising a sample separation filter positioned between the needle guide sheath and the sample container engagement member, wherein the sample separation filter optionally is detachable from the needle guide.
16. The needle guide of clause 20, wherein the sample separation filter comprises a filter selected from the group consisting of a mesh filter, a shaped surface allowing for fluid run-on into the sample collection container, and a folded filter paper.
17. A method of retrieving a sample from a biopsy needle, comprising:
   (a) positioning a biopsy needle containing a biopsy sample at a distal region of a biopsy needle cannula into a biopsy needle mounting fixture on a top surface of a base element, wherein the top surface of the base element further comprises a collection container mounting fixture and a plurality of receptacles, wherein the biopsy needle is accommodated in one of the plurality of receptacles and wherein a needle guide is accommodated in another of the plurality of receptacles;
   (b) positioning a sample collection container in the collection container mounting fixture;
   (c) positioning a sample container engagement member of a needle guide on the sample collection container; wherein the needle guide comprises:
      an elongated member having an interior cavity extending from an open upper end to an open lower end;
      a needle guide capping member positioned over the open upper end, the needle guide capping member having an access port;
      a sample retrieval dart positioned in the interior cavity near the open upper end,
      and comprising a dart body having a dart lumen providing access between the open upper end and the interior cavity at the open lower end, wherein the dart lumen is in-line with the access port;
      a non-return valve positioned within the dart body in line with the dart lumen, and
      a needle guide sheath positioned in the dart lumen below the non-return valve and
      extending downwards from the dart body into the interior cavity at the lower end,
      wherein the open lower end comprises the sample container engagement member;
   (c) inserting a distal end of a sheath of the biopsy needle into the access port of the needle guide;
   (d) positioning the distal end of the sheath of the biopsy needle at the non-return valve;
   (e) extending a distal portion of the biopsy needle cannula into the needle guide sheath; and
   (f) expelling at least a portion of the sample from the biopsy needle cannula into the sample collection container; wherein the expelling comprises at least one of applying a positive pressure to a proximal end of the biopsy needle cannula and applying a vacuum to a distal end of the biopsy needle cannula.
18. The method of clause 17, wherein the sample comprises a histology portion and a cytology portion.
19. The method of clause 18, wherein the needle guide further comprises a sample separation filter positioned between the needle guide sheath and the sample container engagement member.
20. The method of clause 19, wherein the sample separation filter is detachable from the needle guide.
21. The method of clause 19, wherein the sample separation filter comprises a filter selected from the group consisting of a mesh filter, a shaped surface allowing for fluid run-on into the sample collection container, and a folded filter paper.
22. The method of clause 18, wherein the histology portion is captured on the sample separation filter and wherein the cytology portion is captured in the sample collection container.

## Claims

1. A kit comprising:
(a) a biopsy needle;
(b) a needle guide; and
(b) a container comprising
a base element comprising a top surface provided with a biopsy needle mounting fixture, a collection container mounting fixture and a plurality of receptacles, wherein the top surface comprises a front edge, a back edge,
a first side wall and a second side wall each extending upwards from the top surface; and
a cover hinged to the back edge and shaped to extend over the first and second side walls to the front edge and to close the top surface,
wherein the biopsy needle is accommodated in one of the plurality of receptacles and wherein the needle guide is accommodated in another of the plurality of receptacles.

2. The kit of claim 1, wherein the first and the second side walls each slope towards the front and the back edge of the base element and wherein a sealing lip for engaging the cover is positioned on the front edge.

3. The kit of claim 1 or 2, wherein the needle guide comprises:
an elongated member having an interior cavity extending from an open upper end to an open lower end;
a needle guide capping member positioned over the open upper end, the needle guide capping member having an access port;
a sample retrieval dart positioned within the interior cavity near the open upper end, and comprising a dart body having a dart lumen providing access between the open upper end and the interior cavity at the open lower end, wherein the dart lumen is in-line with the access port;
a non-return valve positioned within the dart body, in-line with the dart lumen, and a needle guide sheath positioned in the dart lumen below the non-return valve and extending downwards from the dart body into the interior cavity at the lower end, wherein the open lower end comprises a sample container engagement member.

4. The kit of claim 1, 2, or 3, further comprising a biopsy needle sheath catch positioned on the top surface.

5. The kit of any preceding claim, further comprising a pump station comprising:
a syringe mounting fixture, and
a syringe plunger activation unit comprising a activation member movable to engage a plunger of a syringe mounted in the syringe mounting fixture and to move the plunger to expel fluid from a needle hub of the syringe, and a handle operatively connected to the activation member.

6. The kit of any preceding claim, further comprising at least one component selected from the group consisting of: a vacuum syringe, a wash syringe, a collection container and a dislodge tool, wherein the at least one component is accommodated in the plurality of receptacles.

7. The kit of any preceding claim, wherein the biopsy needle mounting fixture is sized and located to accept a handle of the biopsy needle and to position a proximal end of the biopsy needle handle beyond the front edge of the base element.

8. The kit of any preceding claim, further comprising a sample collection container having a base sized to engage the collection container mounting fixture and a top opening sized to engage the sample container engagement member of the needle guide, optionally wherein the sample collection container further comprises a sample separation filter positioned between the base and the top opening, said sample separation filter optionally being detachable from the sample collection containe, further optionally wherein the sample separation filter comprises a filter selected from the group consisting of a mesh filter, a shaped surface allowing for fluid run-on into the sample collection container, and a folded filter paper.

9. The kit of any preceding claim, further comprising a centrifuge container mounting fixture on the top surface.

10. The kit of any preceding claim, wherein the needle guide further comprises a sample separation filter positioned between the needle guide sheath and the sample container engagement member, and further comprising a sample collection container, wherein the sample separation filter optionally is detachable from the needle guide, optionally, wherein the sample separation filter comprises a filter selected from the group consisting of a mesh filter, a shaped surface allowing for fluid run-on into the sample collection container, and a folded filter paper.

11. A needle guide comprising:
an elongated member having an interior cavity extending from an open upper end to an open lower end;
a needle guide capping member positioned over the open upper end, the needle guide capping member having an access port;
a sample retrieval dart positioned in the interior cavity near the open upper end, and comprising a dart body having a dart lumen providing access between the open upper end and the interior cavity at the open lower end, wherein the dart lumen is in-line with the access port;
a non-return valve positioned within the dart body in line with the dart lumen, and a needle guide sheath positioned in the dart lumen below the non-return valve and extending downwards from the dart body into the interior cavity at the lower end, wherein the open lower end comprises a sample container engagement member.

12. The needle guide of claim 11, further comprising a sample separation filter positioned between the needle guide sheath and the sample container engagement member, wherein the sample separation filter optionally is detachable from the needle guide, further optionally wherein the sample separation filter comprises a filter selected from the group consisting of a mesh filter, a shaped surface allowing for fluid run-on into the sample collection container, and a folded filter paper.

13. A method of retrieving a sample from a biopsy needle, comprising:
(a) positioning a biopsy needle containing a biopsy sample at a distal region of a biopsy needle cannula into a biopsy needle mounting fixture on a top surface of a base element, wherein the top surface of the base element further comprises a collection container mounting fixture and a plurality of receptacles, wherein the biopsy needle is accommodated in one of the plurality of receptacles and wherein a needle guide is accommodated in another of the plurality of receptacles;
(b) positioning a sample collection container in the collection container mounting fixture;
(c) positioning a sample container engagement member of a needle guide on the sample collection container; wherein the needle guide comprises:
an elongated member having an interior cavity extending from an open upper end to an open lower end;
a needle guide capping member positioned over the open upper end, the needle guide capping member having an access port;
a sample retrieval dart positioned in the interior cavity near the open upper end, and comprising a dart body having a dart lumen providing access between the open upper end and the interior cavity at the open lower end, wherein the dart lumen is in-line with the access port;
a non-return valve positioned within the dart body in line with the dart lumen, and
a needle guide sheath positioned in the dart lumen below the non-return valve and extending downwards from the dart body into the interior cavity at the lower end, wherein the open lower end comprises the sample container engagement member;
(c) inserting a distal end of a sheath of the biopsy needle into the access port of the needle guide;
(d) positioning the distal end of the sheath of the biopsy needle at the non-return valve;
(e) extending a distal portion of the biopsy needle cannula into the needle guide sheath; and
(f) expelling at least a portion of the sample from the biopsy needle cannula into the sample collection container; wherein the expelling comprises at least one of applying a positive pressure to a proximal end of the biopsy needle cannula and applying a vacuum to a distal end of the biopsy needle cannula.

14. The method of claim 13, wherein the sample comprises a histology portion and a cytology portion, optionally wherein the needle guide further comprises a sample separation filter positioned between the needle guide sheath and the sample container engagement member, further optionally wherein the sample separation filter is detachable from the needle guide and further optionally wherein the sample separation filter comprises a filter selected from the group consisting of a mesh filter, a shaped surface allowing for fluid run-on into the sample collection container, and a folded filter paper.

15. The method of claim 13 or 14, wherein the histology portion is captured on the sample separation filter and wherein the cytology portion is captured in the sample collection container.
